(19) **Europäisches Patentamt / European Patent Office / Office européen des brevets**

(11) **EP 4 006 147 A1**

(12) # EUROPEAN PATENT APPLICATION
published in accordance with Art. 153(4) EPC

(43) Date of publication:
**01.06.2022 Bulletin 2022/22**

(21) Application number: **20847094.8**

(22) Date of filing: **21.07.2020**

(51) International Patent Classification (IPC):
**C12N 5/10** (2006.01)  **C12N 1/15** (2006.01)
**C12N 1/19** (2006.01)  **C12N 1/21** (2006.01)
**C12N 15/54** (2006.01)  **C12N 15/63** (2006.01)
**C12N 9/00** (2006.01)  **C12N 9/10** (2006.01)

(52) Cooperative Patent Classification (CPC):
**C12N 5/10; C12N 9/00; C12N 9/10; C12N 15/63**

(86) International application number:
**PCT/JP2020/028319**

(87) International publication number:
**WO 2021/020245 (04.02.2021 Gazette 2021/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.07.2019 JP 2019137768**

(71) Applicant: **TOYOBO CO., LTD.**
**Osaka-shi**
**Osaka 530-8230 (JP)**

(72) Inventors:
- **HIYAMA, Takayoshi**
  **Osaka-shi, Osaka 530-8230 (JP)**
- **YOKOE, Sho**
  **Tsuruga-shi, Fukui 914-8550 (JP)**

(74) Representative: **Vossius & Partner**
**Patentanwälte Rechtsanwälte mbB**
**Siebertstraße 3**
**81675 München (DE)**

(54) **VARIANT REVERSE TRANSCRIPTASE EXHIBITING EXCELLENT STABILITY**

(57)    An object is to provide a novel variant reverse transcriptase with excellent stability. The present invention provides a variant reverse transcriptase consisting of an amino acid sequence having at least 90% identity with an amino acid sequence represented by SEQ ID NO: 1, wherein a cysteine residue at a position corresponding to at least one position selected from the group consisting of 90, 157, 236, 262, 310, 409, 495, and 635 is modified. In a specific embodiment, the modification of the cysteine residue is substitution with at least one non-polar amino acid residue selected from the group consisting of alanine, glycine, valine, leucine, and isoleucine.

EP 4 006 147 A1

**Description**

Technical Field

The present invention relates to a variant reverse transcriptase. More specifically, the present invention relates to a variant reverse transcriptase with excellent storage stability over time and thermal stability, a reverse transcription method using the variant reverse transcriptase, a polynucleotide encoding the variant reverse transcriptase, a kit comprising the variant reverse transcriptase, and the like.

Background Art

Reverse transcriptases generally have an activity of synthesizing cDNA using RNA as a template (hereinafter referred to as "RNA-dependent DNA polymerase activity") and an activity of degrading RNA strands of RNA/DNA hybrids (hereinafter referred to as "RNase H activity"). Reverse transcriptases are used, for example, for the analysis of the base sequence of mRNA that directly reflects the amino acid sequence of protein expressed in the living body, the construction of cDNA libraries, RT-PCR, and the like. Moloney murine leukemia virus reverse transcriptase (MMLV), avian myeloblastosis virus reverse transcriptase (AMV), etc., are conventionally known as reverse transcriptases used for such purposes.

If mRNA has a base sequence that tends to form secondary structures, the secondary structures interfere with cDNA synthesis by reverse transcriptases. Accordingly, it is desirable to synthesize cDNA while suppressing the formation of secondary structures by raising the reaction temperature. However, the Moloney murine leukemia virus reverse transcriptase and avian myeloblastosis virus reverse transcriptase often have low thermal stability, and may be inactivated at high temperatures at which the formation of RNA secondary structures is suppressed. Therefore, in recent years, various variant reverse transcriptases having higher thermal stability than wild-type reverse transcriptase and having improved reactivity even at 42 to 60°C, at which stability is generally low, have been developed, for example, by introducing multiple amino acid mutations into reverse transcriptases (PTL 1, PTL 2, and NPL 1).

Moreover, reverse transcriptases are known to have low storage stability, and may be gradually inactivated during storage not only at high temperatures at which the formation of RNA secondary structures is suppressed, but also under relatively mild conditions (e.g., 4°C and 25°C), thereby losing their enzyme activity. Therefore, strict temperature control and expiration date control during storage and transportation are required.

For the reasons stated above, the development of a novel and more useful reverse transcriptase with improved thermal stability and/or stability over time during storage compared with conventional ones has been long awaited.

Citation List

Patent Literature

PTL 1: JP2000-139457A
PTL 2: JP6180002B

Non-patent Literature

NPL 1: Journal of Biotechnology, Vol. 150, Issue 3, pp. 299-306 (published in 2010)

Summary of Invention

Technical Problem

The present invention was made in view of the above prior art. An object of the present invention is to provide a novel and more useful variant reverse transcriptase with improved storage stability over time and/or thermal stability.

Solution to Problem

As a result of intensive studies in view of the above problem, the present inventors found that a reverse transcriptase with improved storage stability over time and/or thermal stability can be obtained by modifying a cysteine residue in a specific site of the reverse transcriptase, thereby arriving at the present invention.

Specifically, the typical present invention includes the following configurations.

[Item 1]

A variant reverse transcriptase consisting of an amino acid sequence having at least 90% identity with an amino acid sequence represented by SEQ ID NO: 1, wherein a cysteine residue at a position corresponding to at least one position selected from the group consisting of 90, 157, 236, 262, 310, 409, 495, and 635 is modified.

[Item 2]

The variant reverse transcriptase according to Item 1, consisting of an amino acid sequence having deletion, substitution, and/or addition of one or several amino acid residues in the amino acid sequence represented by SEQ ID NO: 1, wherein a cysteine residue at a position corresponding to at least one position selected from the group consisting of 90, 157, 236, 262, 310, 409, 495, and 635 is modified.

[Item 3]

The variant reverse transcriptase according to Item 1 or 2, wherein the modification of the cysteine residue is deletion and/or substitution of the cysteine residue with another amino acid residue.

[Item 4]

The variant reverse transcriptase according to any one of Items 1 to 3, wherein the modification of the cysteine residue is substitution with at least one non-polar amino acid residue selected from the group consisting of alanine, glycine, valine, leucine, and isoleucine.

[Item 5]

The variant reverse transcriptase according to any one of Items 1 to 4, wherein the modification of the cysteine residue is substitution with an alanine residue.

[Item 6]

The variant reverse transcriptase according to any one of Items 1 to 5, wherein in the amino acid sequence having at least 90% identity with the amino acid sequence represented by SEQ ID NO: 1, at least a cysteine residue at a position corresponding to position 310 is modified.

[Item 7]

The variant reverse transcriptase according to any one of Items 1 to 6, wherein the modification of the cysteine residue at the position corresponding to position 310 is substitution with a non-polar amino acid residue selected from the group consisting of alanine, glycine, valine, leucine, and isoleucine.

[Item 8]

The variant reverse transcriptase according to any one of Items 1 to 7, wherein the modification of the cysteine residue at the position corresponding to position 310 is substitution with an alanine residue.

[Item 9]

The variant reverse transcriptase according to any one of Items 1 to 8, which lacks RNase activity.

[Item 10]

A variant reverse transcriptase having a residual activity of 30% or more when stored at 25°C for 35 days.

[Item 11]

A variant reverse transcriptase having a residual activity of 50% or more when stored at 25°C for 35 days.

[Item 12]

A polynucleotide encoding the reverse transcriptase according to any one of Items 1 to 8.

[Item 13]

A vector comprising the polynucleotide according to Item 12.

[Item 14]

A cell transformed with the vector according to Item 13.

[Item 15]

A reagent comprising at least one selected from the group consisting of the variant reverse transcriptase according to any one of Items 1 to 11, the polynucleotide according to Item 12, the vector according to Item 13, and the cell according to Item 14.

[Item 16]

A method for producing the variant reverse transcriptase according to any one of Items 1 to 11 using at least one selected from the group consisting of the polynucleotide according to Item 12, the vector according to Item 13, the cell according to Item 14, and the reagent according to Item 15.

[Item 17]

A reverse transcription method comprising synthesizing cDNA from an RNA template using the variant reverse transcriptase according to any one of Items 1 to 11.

[Item 18]

A kit comprising the variant reverse transcriptase according to any one of Items 1 to 11.

[Item 19]

The kit according to Item 18, for use in synthesis of cDNA using RNA as a template.

Advantageous Effects of Invention

The present invention provides a novel and useful variant reverse transcriptase with improved storage stability over time and/or thermal stability. Due to the excellent storage stability over time, the variant reverse transcriptase of the present invention can be more easily handled during storage and transportation, and can be used as a highly convenient reagent. Further, due to the excellent thermal stability, the variant reverse transcriptase of the present invention allows efficient reverse transcription reactions, for example, in a nucleic acid synthesis method including a step of raising the reaction temperature.

Brief Description of Drawings

Fig. 1 shows the results of a stability test of wild-type reverse transcriptase (WT) in Example 4.
Fig. 2 shows the results of a stability test of the variant reverse transcriptase (C310A) of the present invention in Example 4.

Description of Embodiments

The present invention is described in detail below; however, the present invention is not limited thereto.

The variant reverse transcriptase of the present invention is characterized in that a cysteine residue at a specific position of wild-type reverse transcriptase is modified. The present invention is based on the novel finding that a reverse transcriptase with improved storage stability over time and excellent thermal stability can be obtained by modifying a cysteine residue. Cysteine has a thiol group and generally contributes to the maintenance of the three-dimensional structure of protein through the formation of disulfide bonds. The present invention is intended to modify such a cysteine residue (e.g., deletion and/or substitution with another amino acid), thereby changing the performance of the reverse transcriptase and improving thermal stability and/or storage stability.

Therefore, in the present invention, a cysteine residue at a specific position of wild-type reverse transcriptase may be modified, and the mode of modification of the cysteine residue is not limited as long as the effects of the present invention can be achieved. For example, the modification of the cysteine residue can be deletion and/or substitution of the cysteine residue with another amino acid. From the viewpoint of more reliably achieving a high stabilization effect, substitution with another amino acid residue is preferable, and substitution with a non-polar amino acid residue is particularly preferable. The variant reverse transcriptase of the present invention as described above characteristically has reverse transcription activity and improved thermal stability and/or storage stability compared with the reverse transcriptase before modification.

In the present invention, "wild-type reverse transcriptase" (hereinafter also referred to as "WT") refers to a reverse transcriptase into which no mutation is artificially introduced. Examples of the wild-type reverse transcriptase include a reverse transcriptase comprising the amino acid sequence represented by SEQ ID NO: 1. The "amino acid sequence represented by SEQ ID NO: 1" refers to the amino acid sequence (Moloney murine leukemia virus reverse transcriptase) represented by SEQ ID NO: 1 shown in the sequence listing. Further, "Moloney murine leukemia virus reverse transcriptase" is also expressed as "MMLV reverse transcriptase." The present invention can be a variant reverse transcriptase derived from the amino acid sequence of Moloney murine leukemia virus reverse transcriptase, or may be a variant reverse transcriptase derived from a protein (e.g., ortholog or homolog) comprising an amino acid sequence with high homology to the amino acid sequence represented by SEQ ID NO: 1 and in a predetermined relationship to the amino acid sequence represented by SEQ ID NO: 1 in terms of the amino acid sequence identity etc. From the viewpoint of more reliably achieving a higher stability improvement effect, a variant reverse transcriptase derived from the amino acid sequence of Moloney murine leukemia virus reverse transcriptase is preferred.

In one embodiment, the variant reverse transcriptase of the present invention is characterized in that in the amino acid sequence represented by SEQ ID NO: 1, a cysteine residue at a position corresponding to at least one position selected from the group consisting of 90, 157, 236, 262, 310, 409, 495, and 635 is modified. The amino acid sequence before modification is not limited to one that is completely identical to SEQ ID NO: 1, and is not particularly limited as long as the reverse transcription activity is not lost. For example, the amino acid sequence has at least 90%, preferably at least 95%, more preferably at least 96%, even more preferably at least 97%, still even more preferably at least 98%, and particularly preferably at least 99%, identity with the amino acid sequence represented by SEQ ID NO: 1. The amino acid sequence identity can be evaluated by any method known in the art. For example, the amino acid sequence identity can be calculated using a commercially available analysis tool or an analysis tool available through a telecommunication line (Internet). For example, the amino acid sequence identity can be calculated using the default parameters of the National Center for Biotechnology Information (NCBI) homology algorithm BLAST (Basic Local Alignment Search Tool; http://www.ncbi.nlm.nih.gov/BLAST/). Further, the amino acid sequence before modification may be an amino acid sequence having deletion, substitution, and/or addition of one or several amino acids in the amino acid sequence represented by SEQ ID NO: 1. The number of amino acids indicated by "one or several" is not particularly limited as long as the reverse transcription activity is not lost. For example, "one or several" refers to 1 to 20, preferably 1 to 10, more preferably 1 to 5, and even more preferably 1 to 3. The amino acid sequence before modification as described above may be, for example, artificially produced by a genetic engineering method, or may be an amino acid sequence of a naturally occurring protein.

In the present specification, simplified symbols in alphabetical notation may be used for base sequences, amino acid sequences, and their individual constituents; however, all follow the practice in the fields of molecular biology and gene engineering. Further, in the present specification, expressions such as "C90A" are used to briefly indicate mutations in the amino acid sequence. "C90A" indicates substitution of 90th cysteine with alanine; that is, it shows the type and location of amino acid residue before substitution, and the type of amino acid residue after substitution. Further, SEQ ID NOs correspond to SEQ ID NOs shown in the sequence listing unless otherwise specified. In the case of a multiple variant, the above expressions are expressed by connection with "/." In the present specification, in an amino acid sequence that is not completely identical to the amino acid sequence represented by SEQ ID NO: 1, the site corresponding to a certain position (order) on SEQ ID NO: 1 refers to the position corresponding to this position of SEQ ID NO: 1 when the primary structures of the sequences are compared (aligned).

Moreover, in the present specification, the terms "variant" and "modified" in "variant reverse transcriptase" and "modified reverse transcriptase" are used interchangeably, and mean that they have an amino acid sequence different from conventionally known reverse transcriptases; these terms are not used to distinguish between artificial mutations and natural mutations. Therefore, the variant reverse transcriptase of the present invention refers to a reverse transcriptase having an amino acid sequence different from SEQ ID NO: 1, obtained by modifying one or more amino acids in the amino acid sequence represented by SEQ ID NO: 1. It does not matter whether the variant reverse transcriptase is a variant reverse transcriptase obtained by an artificial mutation or a variant reverse transcriptase resulting from a naturally occurring mutation.

In a specific preferred embodiment, the reverse transcriptase of the present invention is obtained by modifying a cysteine residue at a position corresponding to at least one position selected from the group consisting of 90, 157, 236, 262, 310, 409, 495, and 635 in the amino acid sequence represented by SEQ ID NO: 1 or an amino acid sequence in a predetermined relationship to the amino acid sequence represented by SEQ ID NO: 1 in terms of the amino acid sequence identity etc. Therefore, as long as the effects of the present invention can be achieved, the variant reverse transcriptase of the present invention may be one obtained by modifying cysteine residues at positions corresponding to two or more, three or more, four or more, five or more, six or more, or seven or more, of the above positions in the amino acid sequence mentioned above; or may be one obtained by modifying cysteine residues at positions corresponding to all of the eight positions specified above. As shown in the results of Examples, provided later, a particularly high stabilization effect is achieved by modifying a cysteine residue at a position corresponding to position 310 of SEQ ID NO: 1. Therefore, in a specific preferred embodiment, at least a cysteine residue at a position corresponding to position 310 in the above-mentioned amino acid sequence can be modified.

In a specific preferred embodiment, the variant reverse transcriptase of the present invention is obtained by substituting, with a non-polar amino acid, a cysteine residue at a position corresponding to at least one position selected from the group consisting of 90, 157, 236, 262, 310, 409, 495, and 635 in the amino acid sequence represented by SEQ ID NO: 1 or an amino acid sequence in a predetermined relationship to the amino acid sequence represented by SEQ ID NO: 1 in terms of the amino acid sequence identity etc. Preferred is one in which a cysteine residue at a position corresponding to at least one position selected from the group consisting of 90, 157, 236, 262, 310, 409, 495, and 635 is substituted with glycine, alanine, isoleucine, leucine, valine, phenylalanine, proline, methionine, or tryptophan; more preferred is one in which the above cysteine residue is substituted with glycine, alanine, isoleucine, leucine, valine, phenylalanine, or proline; even more preferred is one in which the above cysteine residue is substituted with glycine, alanine, isoleucine, leucine, or valine; still even more preferred is one in which the above cysteine residue is substituted with glycine, alanine, or isoleucine; and particularly preferred is one in which the above cysteine residue is substituted with alanine or isoleucine. Glycine, alanine, isoleucine, leucine, and valine are non-polar amino acids and are known to have an isoelectric point of about 6.0; they can be expected to exhibit the same effect as amino acids that can exhibit common properties.

The variant reverse transcriptase of the present invention can be obtained, for example, by producing a protein having the amino acid sequence represented by SEQ ID NO: 1 or an amino acid sequence in a predetermined relationship to the amino acid sequence represented by SEQ ID NO: 1 in terms of the amino acid sequence identity etc., in which a cysteine residue at a position corresponding to at least one position selected from the group consisting of 90, 157, 236, 262, 310, 409, 495, and 635 is modified. The method for introducing an amino acid modification in the amino acid sequence could have been suitably carried out by a person skilled in the art by any method known in the art. For example, a new variant reverse transcriptase protein can be produced through protein engineering technique by introducing a mutation into a gene encoding wild-type reverse transcriptase to introduce an amino acid modification at a predetermined position. As one aspect of the method for introducing an amino acid modification, site-specific mutagenesis based on the inverse PCR method can be used. For example, a KOD-Plus-Mutagenesis Kit (produced by Toyobo Co., Ltd.) can be used to obtain a transformant containing a plasmid into which the desired mutation has been introduced, by (1) modifying a plasmid into which the target gene has been inserted, annealing the plasmid with a mutagenic primer, and then performing an extension reaction using KOD DNA polymerase, (2) repeating the cycle of (1) 15 times, (3) selectively cleaving only the plasmid used as a template using the restriction enzyme DpnI, (4) cyclizing the newly synthesized gene by phosphorylation and ligation, and (5) transforming the cyclized gene into *Escherichia coli.* This kit can be preferably used for the production of the variant reverse transcriptase of the present invention.

In a specific embodiment, the variant reverse transcriptase of the present invention may be one that lacks RNase activity. Examples of variant reverse transcriptases lacking RNase activity include, but are not limited to, those in which aspartic acid at a position corresponding to position 524 is substituted with alanine. The RNase activity of wild-type reverse transcriptase may degrade RNA, which is a template for the reverse transcription reaction. In particular, this activity can be a problem in the synthesis of cDNA using long-chain RNA (e.g., full-length RNA) as a template. Variant reverse transcriptases modified to lack RNase activity are preferable because in a reverse transcription reaction using a long-chain RNA as a template, it is possible to suppress the degradation of the RNA strand template during the reaction.

As shown in Test Examples, provided later, the present invention can provide a variant reverse transcriptase with improved storage stability and/or thermal stability. In a preferred embodiment, the variant reverse transcriptase provided by the present invention is excellent in both storage stability and thermal stability.

Specifically, the variant reverse transcriptase with excellent storage stability over time provided by the present invention can be, for example, a variant reverse transcriptase having a residual activity of 20% or more when stored at 25°C for 35 days, and preferably a variant reverse transcriptase having a residual activity of 30% or more, more preferably 40% or more, and even more preferably 50% or more. Further, the variant reverse transcriptase with excellent storage stability over time according to the present invention can be, for example, a variant reverse transcriptase having a residual activity of 20% or more, preferably 30% or more, more preferably 40% or more, and even more preferably 50% or more, when stored at 25°C for 25 days. Moreover, the variant reverse transcriptase with excellent storage stability over time according to the present invention can be, for example, a variant reverse transcriptase having a residual activity of 40% or more, preferably 45% or more, more preferably 50% or more, and even more preferably 60% or more, when stored at 25°C for 10 days. Variant reverse transcriptases having such a high residual activity after long-term storage could have been suitably obtained by a person skilled in the art, for example, by modifying one or more amino acids in the amino acid sequence represented by SEQ ID NO: 1 or an amino acid sequence in a predetermined relationship to the amino acid sequence represented by SEQ ID NO: 1 in terms of the amino acid sequence identity etc., and evaluating the obtained variant reverse transcriptases by measurement of the residual activity as described later.

In a specific embodiment, the variant reverse transcriptase with excellent storage stability over time provided by the present invention can be, for example, a variant reverse transcriptase having a higher residual activity than wild-type reverse transcriptase when stored at 25°C for 10 days, 25 days, or 35 days. A specific example can be a variant reverse transcriptase having a residual activity about 1.1 times or more, preferably about 1.2 times or more, more preferably 1.3 times or more, and even more preferably about 1.4 times or more, higher than that of wild-type reverse transcriptase when stored at 25°C for 10 days. Another example can be a variant reverse transcriptase having a residual activity about 1.1 times or more, preferably about 1.5 times or more, more preferably about 2 times or more, and particularly preferably 4 times or more, higher than that of wild-type reverse transcriptase when stored at 25°C for 25 days. Still another example can be a variant reverse transcriptase having a residual activity about 1.1 times or more, preferably about 1.5 times or more, more preferably about 2 times or more, and particularly preferably about 4 times or more, higher than that of wild-type reverse transcriptase when stored at 25°C for 35 days. Variant reverse transcriptases having such a high residual activity after long-term storage at 25°C could have been suitably obtained by a person skilled in the art by evaluating both wild-type reverse transcriptase and a variant reverse transcriptase with one or more modified amino acid residues by measurement of the residual activity as described later, and comparing the residual activity of the variant reverse transcriptase with the residual activity of the wild-type reverse transcriptase.

In a further embodiment, the variant reverse transcriptase with excellent thermal stability provided by the present invention can be, for example, a variant reverse transcriptase having a residual activity of 70% or more when heat-treated at 45°C for 5 minutes. Further, the variant reverse transcriptase with excellent thermal stability according to the present invention can be, for example, a variant reverse transcriptase having a residual activity of 65% or more even when heat-treated at 45°C for 10 minutes. Furthermore, the variant reverse transcriptase with excellent thermal stability according to the present invention may be, for example, a variant reverse transcriptase having a residual activity of 65% or more, and preferably 68% or more, when heat-treated at 50°C for 5 minutes. Moreover, the variant reverse transcriptase with excellent thermal stability according to the present invention may be, for example, a variant reverse transcriptase having a residual activity of 20% or more, and preferably 25% or more, when heat-treated at 55°C for 5 minutes. Variant reverse transcriptases having such a high residual activity after heat treatment could have been suitably obtained by a person skilled in the art, for example, by modifying one or more amino acids in the amino acid sequence represented by SEQ ID NO: 1 or an amino acid sequence in a predetermined relationship to the amino acid sequence represented by SEQ ID NO: 1 in terms of the amino acid sequence identity etc., and evaluating the obtained variant reverse transcriptases by measurement of the residual activity as described later.

In a specific embodiment, the variant reverse transcriptase with excellent thermal stability provided by the present invention can be, for example, a variant reverse transcriptase having a higher residual activity than wild-type reverse transcriptase when heat-treated at 45°C for 5 minutes, at 50°C for 5 minutes, or at 55°C for 5 minutes. A specific example can be a variant reverse transcriptase having a residual activity about 1.1 times or more, preferably about 1.2 times or more, more preferably 1.3 times or more, and even more preferably about 1.4 times or more, higher than that of wild-type reverse transcriptase when heat-treated at 45°C for 5 minutes. Another example can be a variant reverse transcriptase having a residual activity about 1.1 times or more, and preferably about 1.2 times or more, higher than that of wild-type reverse transcriptase when heat-treated at 50°C for 5 minutes. Still another example can be a variant reverse transcriptase having a residual activity about 1.1 times or more, preferably about 1.2 times or more, more preferably about 1.4 times or more, and particularly preferably about 1.6 times or more, higher than that of wild-type reverse transcriptase when heat-treated at 55°C for 5 minutes. Variant reverse transcriptases having such a high residual activity after heat treatment could have been suitably obtained by a person skilled in the art, for example, by evaluating both wild-type reverse transcriptase and a variant reverse transcriptase with one or more modified amino acid residues by measurement of the residual activity as described later, and comparing the residual activity of the variant reverse transcriptase with the residual activity of the wild-type reverse transcriptase.

Method for Measuring Reverse Transcription Activity

In the present specification, the reverse transcription activity of reverse transcriptases can be measured by the following procedure. In this measurement method, when the enzyme activity is high, a sample containing the measurement target may be suitably diluted for the measurement.

First, 10 μL of A liquid, 22 μL of B liquid, and 1 μL of C liquid, all of which are previously prepared as described below, and 12 μL of sterilized water are added to a reaction vessel, such as a microtube, and the mixture is stirred and mixed. Then, 5 μL of a sample solution containing the measurement target or a diluted solution thereof is added and allowed to react at 42°C for 10 minutes. Thereafter, the resultant is cooled, and 150 μL of the following D liquid is added and stirred, and then ice-cooled for 10 minutes. The resulting liquid is filtered through a glass filter (Whatman GF/C filter), and sufficiently washed with 0.1 N hydrochloric acid and ethanol. The radioactivity of the filter is measured using a liquid scintillation counter (Tri-Carb 2810 TR, produced by Packard), and the uptake of nucleotides is measured. One unit of enzyme activity is the amount of enzyme that incorporates 1 nmole of nucleotide into the acid-insoluble fraction per 10 minutes under these conditions.

Reverse Transcriptase Activity Measurement Reagent

- A liquid: 250 mM Tris-HCl (pH: 8.3), 375 mM potassium chloride, 15 mM magnesium chloride, 50 mM dithiothreitol
- B liquid: 1 mg/mL poly A, 1 pmol/μL dT20, 10 mM dTTP
- C liquid: [3H]-dTTP
- D liquid: 0.07 M sodium pyrophosphate, 0.7 M trichloroacetic acid

Measurement of Residual Activity of Reverse Transcription  Activity

The variant reverse transcriptases to be measured are each diluted with a storage buffer (50 mM Tris-HCl (pH: 7.5), 300 mM KCl, 50% glycerol, 0.1 mM EDTA) to 100 U/μL, and the reverse transcription activity value before storage is measured according to the procedure described in the section "Method for Measuring Reverse Transcription Activity" above. Then, the variant reverse transcriptases to be measured diluted with the above storage buffer are stored under specific storage conditions (specifically, storage conditions, for example, in an incubator at 25°C for 10 days to 35 days when storage stability over time is evaluated; and storage conditions, for example, in an incubator at 45°C to 55°C for 5 minutes when thermal stability is evaluated). After a predetermined time has passed from the start of storage (specifically, for example, after 10 days to 35 days when storage stability over time is evaluated; and, for example, after 5 minutes when thermal stability is evaluated), the reverse transcription activity value after storage is measured according to the procedure described in the section "Method for Measuring Reverse Transcription Activity" above, as in the case before storage. Then, the residual activity can be calculated by dividing the reverse transcription activity value after storage by the reverse transcription activity value before storage, as shown in the following formula I.

```
Residual activity (%) = (reverse transcription activity value
after storage/reverse transcription activity value before
storage) × 100 . . . (Formula I)
```

In a further embodiment, the present invention provides a polynucleotide encoding the variant reverse transcriptase of the present invention described above. The polynucleotide encoding the variant reverse transcriptase refers to, for example, a polynucleotide from which the protein of the variant reverse transcriptase of the present invention can be obtained when the polynucleotide is expressed by a conventional method. That is, this polynucleotide refers to a polynucleotide comprising a base sequence corresponding to the amino acid sequence of the protein of the variant reverse transcriptase of the present invention. A person skilled in the art could have easily determined a base sequence corresponding to a predetermined amino acid sequence according to a codon table etc. known in the art. Further, the polynucleotide encoding the variant reverse transcriptase of the present invention also includes a polynucleotide that differs due to codon degeneracy. The polynucleotide can be any nucleic acid polymer, such as DNA or RNA.

In a further embodiment, the present invention provides a vector comprising the polynucleotide. Specifically, the polynucleotide encoding the variant reverse transcriptase is transferred to a vector (e.g., an expression vector or a cloning vector), if necessary. Any vector may be used as long as it allows cloning and/or expression etc. of the variant reverse transcriptase of the present invention. For example, a plasmid can be used. Examples of plasmids include, but are not limited to, pUC118, pUC18, pBR322, pBluescript, pLED-M1, p73, pGW7, pET3a, pET8c, and the like.

In a further embodiment, the present invention provides a cell transformed with the vector. Such a cell can be preferably used to express the protein encoding the variant reverse transcriptase of the present invention. In a specific preferred embodiment, the recombinant host cell of the present invention is obtained by transforming a host cell using the above expression vector. Examples of the host cell include *Escherichia coli* and yeast; *Escherichia coli* is particularly preferred. Examples of *Escherichia coli* include *Escherichia coli* DH5α, JM109, HB101, XL1Blue, PR1, HS641(DE3), BL21(DE3), and the like. That is, it is preferable in the present invention to insert the gene encoding the variant reverse transcriptase into the above vector to obtain an expression vector, and transform the host cell with the expression vector.

In one embodiment, the expression vector of the present invention may contain elements for facilitating the purification of the variant reverse transcriptase, such as extracellular signals and His tags.

A further embodiment provides a method for producing the variant reverse transcriptase using the polynucleotide, the vector, the transformed cell, and/or a reagent containing one or more of them. For example, the host cell is transformed using the expression vector, and then applied to an agar medium containing a drug such as ampicillin to form colonies. The colonies are inoculated into a nutrient medium, such as LB medium or 2 × YT medium, and cultured at 37°C for 12 to 20 hours. Then, the cells are disrupted to extract a crude enzyme solution. Any known method may be used to disrupt the cells. For example, sonication, physical disruption such as French press or glass bead disruption, or lytic enzymes such as lysozyme can be used. Any method may be used to obtain the purified reverse transcriptase from the obtained crude enzyme solution. The variant reverse transcriptase of the present invention can be isolated, for example, by subjecting the crude enzyme solution to centrifugation, ultracentrifugation, ultrafiltration, salting-out, dialysis, ion-exchange column chromatography, adsorption column chromatography, affinity chromatography, gel filtration column chromatography, or the like.

The present invention further provides a reverse transcription method characteristically using the variant reverse transcriptase. The reverse transcription method of the present invention is characterized by synthesizing cDNA from an RNA template using the variant reverse transcriptase of the present invention. The variant reverse transcriptase of the present invention has higher thermal stability than wild-type reverse transcriptase. Accordingly, the reverse transcription method of the present invention allows reverse transcription reactions in a wide temperature range, including temperatures high enough to suppress the formation of RNA secondary structures. Therefore, the reverse transcription method of the present invention is highly versatile because reverse transcription reactions can be performed efficiently regardless of the type of RNA.

In one preferred embodiment, in the reverse transcription method of the present invention, the reverse transcription reaction can be performed by incubating the variant reverse transcriptase, RNA as a template, an oligonucleotide primer complementary to a part of the RNA, and four types of deoxyribonucleoside triphosphates in a reverse transcription reaction buffer.

The reaction temperature in the reverse transcription reaction differs depending on the type of RNA used, the type of variant reverse transcriptase used, etc., and is preferably suitably set according to the type of RNA used, the type of variant reverse transcriptase used, etc. The reaction temperature can be set to 37 to 45°C, for example, when the RNA used does not tend to form secondary structures. Further, for example, when the RNA used tends to form secondary structures, the reaction temperature can be set to a temperature higher than the reaction temperature suitable for wild-type reverse transcriptase, for example, 45 to 60°C. Since the variant reverse transcriptase of the present invention has high thermal stability, there is the advantage that the reverse transcription reaction can be sufficiently performed even under conditions with high reaction temperatures. The reaction time is, for example, about 1 minute to 1 hour, and preferably about 5 minutes to 30 minutes, but is not limited thereto.

The reverse transcription reaction buffer used in the reverse transcription method of the present invention may contain divalent cations, such as magnesium ions and manganese ions. The concentration of divalent cations is preferably suitably set according to the type of variant reverse transcriptase and other components contained in the reverse transcription reaction buffer. For example, the divalent cation concentration of the reverse transcription reaction buffer is set to 1 to 30 mM. Further, the reverse transcription reaction buffer may contain, if necessary, a reducing agent (e.g., dithiothreitol), a stabilizer (e.g., glycerol or trehalose), an organic solvent (e.g., dimethyl sulfoxide or formamide), and other components as long as they do not impair the object of the present invention.

In a further embodiment, the present invention provides a reagent containing the variant reverse transcriptase, the polynucleotide, the vector, and/or the cell. Such a reagent may contain other optional components (e.g., stabilizers, preservatives, and other optional additives) depending on the purpose of use etc. For example, the reagent containing the variant reverse transcriptase may further contain the reverse transcription reaction buffer and the like. The reagent of the present invention is not particularly limited in its use, and can be suitably used, for example, for the reverse transcription reaction that synthesizes cDNA using RNA as a template. Further, the reagent of the present invention can also be preferably used for producing the variant reverse transcriptase of the present invention.

In a further embodiment, the present invention provides a kit comprising the variant reverse transcriptase, the polynucleotide, the vector, the cell, and/or a reagent containing one or more of them. The kit of the present invention can be, for example, a kit for performing the reverse transcription reaction of synthesizing cDNA using RNA as a template, a kit for producing the variant reverse transcriptase of the present invention, or the like, but is preferably a kit for synthesizing cDNA using RNA as a template (this kit is also referred to as "reverse transcription reaction kit" etc.).

In one embodiment, the reverse transcription reaction kit of the present invention is a kit for performing a reverse transcription reaction, and one of its features is that the kit contains the variant reverse transcriptase of the present invention (including a case in which it is provided as a reagent containing the variant reverse transcriptase). Since the reverse transcription reaction kit of the present invention contains the variant reverse transcriptase of the present invention, which has high thermal stability, it can be preferably used even in reverse transcription reactions in a wide temperature range, including temperatures high enough to suppress the formation of RNA secondary structures. In addition, since the kit contains the reverse transcriptase with improved stability during storage, it is easier to manage the kit during transportation and storage than before, and the kit can be suitable for long-term storage. Thus, this kit is highly convenient. The kit of the present invention may further contain, for example, an instruction manual for performing a reverse transcription reaction using the variant reverse transcriptase of the present invention. The kit of the present invention can be provided in the form in which the variant reverse transcriptase etc. are packed, for example, in a single package, and in which information on how to use the kit is included.

In a specific embodiment, for example, in the reverse transcription reaction kit, reagents necessary for performing the reverse transcription reaction may be enclosed in containers different from the container containing the variant reverse transcriptase. If the progress of the reverse transcription reaction during storage of the reagents is stopped, the reagents may be enclosed in the same container as the variant reverse transcriptase. The reagents may be enclosed in a container in amounts suitable for performing the reverse transcription reaction. This eliminates the need to mix the reagents in amounts suitable for the reverse transcription reaction, which facilitates handling.

Examples

The present invention is described in more detail below with reference to Examples. The present invention is not limited to the Examples.

Example 1: Production of MMLV Reverse Transcriptase Plasmids

DNA (SEQ ID NO: 2) encoding Moloney murine leukemia virus-derived reverse transcriptase was cloned into pET-23b(+) to produce a plasmid (pMMLV) into which wild-type MMLV was incorporated. Mutated plasmids were produced using pMMLV as a template and using a KOD-Plus-Mutagenesis Kit (produced by Toyobo Co., Ltd.) by a method according to the instruction manual. Table 1 shows the prepared plasmids and primers used therefor. The obtained plasmids were transformed into BL21-CodonPlus competent cells (Agilent Technologies) and used for enzyme preparation.

Table 1

| Plasmid | Primer |
|---------|--------|
| WT | - |
| C 9 0 A | GCGCAGTCCCCCTGGAACACGCCCC (SEQ ID NO: 3) GGGTACCAGTATTCCCTGGTCCAAC (SEQ ID NO: 4) |
| C 1 5 7 A | GCGCTGAGACTCCACCCCACCAGTC (SEQ ID NO: 5) GAAAAAGGCATCCTTTAAATCAAGC (SEQ ID NO: 6) |
| C 2 3 6 A | GCGCAACAAGGTACTCGGGCCCTGT (SEQ ID NO: 7) GTCTAGCTCAGAAGTGGCGGCCAGC (SEQ ID NO: 8) |
| C 2 6 2 A | GCGCAGAAACAGGTCAAGTATCTGG (SEQ ID NO: 9) AATTTGGGCTTTCTTGGCCGAGGCC (SEQ ID NO: 10) |
| C 3 1 0 A | GCGCGCCTCTGGATCCCTGGGTTTG (SEQ ID NO: 11) GA,4GCCTGCCGTCCCTAGGAACTCC (SEQ ID NO: 12) |

(continued)

| Plasmid | Primer |
|---------|--------|
| C 4 0 9 A | GCGCTACGGATGGTAGCAGCCATTG (SEQ ID NO: 13) AGGGGGCCACCCAGCTGCTACTGGG (SEQ ID NO: 14) |
| C 4 9 5 A | GCGCTTGATATCCTGGCCGAAGCCC (SEQ ID NO: 15) GTTGTGTTGCAGCCCTTCCTCAGGC (SEQ ID NO: 16) |
| C 6 3 5 A | GCGCCAGGACATCAAAAGGGACACA(SEQ ID NO: 17) ATGGATTATGCTAAGTCTTTTGGGC (SEQ ID NO: 18) |

Example 2: Acquisition of Reverse Transcriptase

The cells obtained in Example 1 were cultured as described below. First, 80 mL of sterilized TB medium (Molecular Cloning, 2nd Edition, p.A. 2) containing 100 µg/mL ampicillin was dispensed into a 500-mL Sakaguchi flask. In this medium, a plasmid transformed strain previously cultured at 37°C for 16 hours in 3 mL of LB medium (1% bactotrypton, 0.5% yeast extract, 0.5% sodium chloride) containing 100 µg/mL ampicillin was inoculated and aerobically cultured at 30°C for 16 hours. Then, IPTG (produced by Nacalai Tesque, Inc.) was added to a final concentration of 1 mM, and the cells were aerobically cultured at 30°C for another 4 hours. The cells were collected from the culture medium by centrifugation and suspended in 50 mL of disruption buffer (10 mM Tris-HCl (pH: 7.5), 300 mM KCl, 5% glycerol). Then, the cells were disrupted by sonication to obtain a cell disruption solution. Next, the cell disruption solution was purified with His GraviTrap (GE Healthcare). The washing conditions were 10 mM Tris-HCl (pH: 7.5), 300 mM KCl, 5% glycerol, and 50 mM imidazole. The elution conditions were 10 mM Tris-HCl (pH: 7.5), 300 mM KCl, 5% glycerol, and 300 mM imidazole. Finally, substitution was carried out using a storage buffer (50 mM Tris-HCl (pH: 7.5), 300 mM KCl, 50% glycerol, 0.1 mM EDTA), thereby obtaining each variant reverse transcriptase.

The activity of the reverse transcriptases purified as described above was measured in the following manner. When the enzyme activity was high, the sample was diluted for the measurement.

Reverse Transcriptase Activity Measurement Reagent

A liquid: 250 mM Tris-HCl (pH: 8.3), 375 mM potassium chloride, 15 mM magnesium chloride, 50 mM dithiothreitol
B liquid: 1 mg/mL poly A, 1 pmol/µL dT20, 10 mM dTTP
C liquid: [3H]-dTTP
D liquid: 0.07 M sodium pyrophosphate, 0.7 M trichloroacetic acid

Method for Measuring Reverse Transcriptase Activity

10 µL of A liquid, 22 µL of B liquid, 1 µL of C liquid, and 12 µL of sterilized water were added to a microtube, and the mixture was stirred and mixed. Then, 5 µL of the above purified enzyme diluted solution was added, and allowed to react at 42°C for 10 minutes. Thereafter, the resultant was cooled, and 150 µL of D liquid was added and stirred, and then ice-cooled for 10 minutes. This liquid was filtered through a glass filter (Whatman GF/C filter), and sufficiently washed with 0.1 N hydrochloric acid and ethanol. The radioactivity of the filter was measured using a liquid scintillation counter (Tri-Carb 2810 TR, produced by Packard), and the uptake of nucleotides was measured. One unit of enzyme activity was the amount of enzyme that incorporated 1 nmole of nucleotide into the acid-insoluble fraction per 10 minutes under this condition.

The results of the above measurement confirmed that all of the modified MMLV reverse transcriptases of the present invention had sufficient reverse transcription activity equivalent to that of the wild-type MMLV reverse transcriptase.

Example 3: Stability Test of Variant Reverse Transcriptases

Each variant reverse transcriptase was diluted with a storage buffer to 100 U/µL and stored in an incubator at 25°C. The reverse transcriptase activity of each variant reverse transcriptase was measured 10 days, 25 days, and 35 days after the start of storage. The residual activity was determined by dividing the activity value after storage by the activity value before storage according to the following Formula I.

Residual activity (%) = (reverse transcription activity value
after storage/reverse transcription activity value before
storage) × 100 . . . (Formula I)

Table 2

| | Residual activity | | |
|---|---|---|---|
| | After 10 days | After 25 days | After 35 days |
| WT | 39% | 11% | 8% |
| C90A | 50% | 29% | 24% |
| C157A | 45% | 39% | 30% |
| C262A | 53% | 34% | 29% |
| C236A | 59% | 31% | 24% |
| C310A | 62% | 50% | 50% |
| C409A | 50% | 30% | 28% |
| C495A | 55% | 37% | 29% |
| C635A | 55% | 24% | 24% |

Table 2 shows the residual activity of each variant reverse transcriptase. The wild-type MMLV reverse transcriptase (WT) showed low stability during storage at 25°C, and the activity was reduced to 39% after 10 days and 8% after 35 days. On the other hand, the variant reverse transcriptases obtained by modifying (substituting) cysteine residues at predetermined positions shown in Table 2 with alanine residues showed a residual activity of 45% or more after 10 days and 24% or more even after 35 days. Among the variant reverse transcriptases, particularly C310A significantly contributed to the improvement of stability, and showed a residual activity of 50% even after 35 days. It was indicated that the reverse transcriptases of the present invention had improved stability during storage compared with the wild-type reverse transcriptase. Although the above results show stability during storage at 25°C, it is considered that the stability would be improved even under normally used storage and transportation conditions, such as 4°C and -20°C, which is very useful.

Example 4: Stability Test of Variant Reverse Transcriptases

100 U/μL of variant reverse transcriptase (C310A) prepared in the same manner as in Example 3 and 100 U/μL of wild-type reverse transcriptase (WT) were stored in an incubator at 25°C for 10 days or 30 days. Each reverse transcriptase before and after storage was analyzed by non-reducing SDS-PAGE. 200 U of the variant reverse transcriptase was mixed with a sample buffer (125 mM Tris-HCl (pH: 6.8), 4% (w/v) SDS, 20% glycerol, 0.01% BPB), incubated at 25°C for 1 hour, and then subjected to SDS-PAGE analysis. SuperSep Ace, 10% (FUJIFILM Wako Pure Chemical Corporation) was used for the SDS-PAGE analysis.

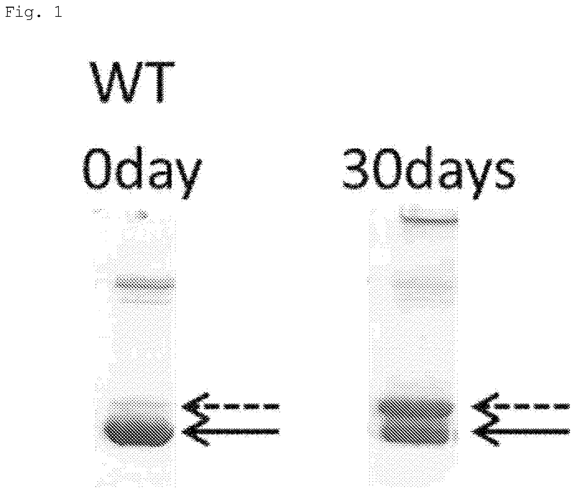

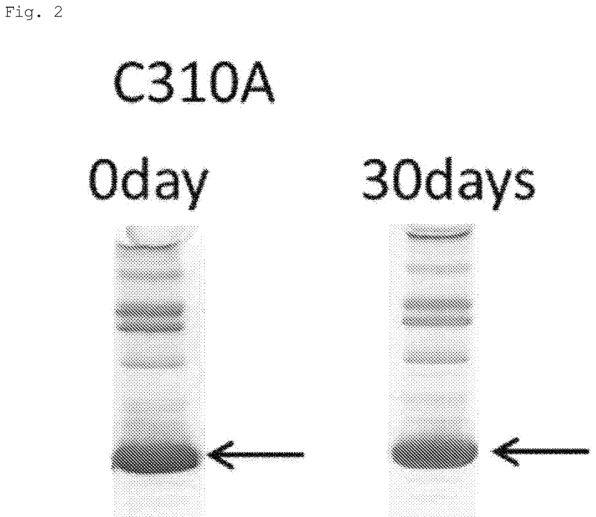

Fig. 1 shows the results of the non-reducing SDS-PAGE analysis of WT before storage ("0 day") and after storage for 30 days ("30 days"), and Fig. 2 shows the results of the non-reducing SDS-PAGE analysis of C310A before storage ("0 day") and after storage for 30 days ("30 days"). The analysis results of WT confirmed a phenomenon in which after incubation for 30 days at 25°C, the band (indicated by the solid arrow in the figure) of the reverse transcriptase faded, and the upper band (indicated by the dotted arrow in the figure) of the reverse transcriptase darkened. This is due to the degeneration and deactivation of the reverse transcriptase during storage. In contrast, the analysis results of the C310A variant reverse transcriptase revealed that an upper band (band at the position corresponding to the dotted arrow in Fig. 1) did not appear in the C310A variant reverse transcriptase. The results also indicate that C310A had improved stability during storage.

Example 5: Heat Resistance Test of Variant Reverse Transcriptases

Various variant reverse transcriptases (C90A, C157A, C236A, C262A, C409A, C495A, and C635A) were each diluted with a storage buffer (50 mM Tris-HCl (pH: 7.5), 300 mM KCl, 50% glycerol, 0.1 mM EDTA) to 10 U/μL, and heat-treated at 45°C for 5 minutes. Then, the reverse transcription activity was measured to determine the residual activity of each variant reverse transcriptase after heat treatment. C310A variant reverse transcriptase was heat-treated at 50°C or 55°C for 5 minutes, in addition to heat treatment at 45°C for 5 minutes, and the residual activity was similarly measured. The residual activity was determined by dividing the measured activity after heat treatment by the measured activity before heat treatment.

Table 3

| Name | Treatment conditions | Residual activity |
|------|---------------------|-------------------|
| WT | | 65% |
| C90A | | 96% |
| C157A | | 82% |
| C236A | | 91% |
| C262A | 45°C, 5 min | 85% |
| C409A | | 95% |
| C495A | | 78% |
| C635A | | 85% |

Table 4

| Name | Treatment conditions | Residual activity |
|------|---------------------|-------------------|
| WT | 45°C, 5 min | 65% |
| | 50°C, 5 min | 60% |
| | 55°C, 5 min | 17% |
| C310A | 45°C, 5 min | 93% |
| | 50°C, 5 min | 70% |
| | 55°C, 5 min | 29% |

According to Table 3, WT showed a residual activity of 65% after heat treatment at 45°C for 5 minutes, whereas all of the variant reverse transcriptases showed a very high residual activity of about 80% to 95%. This revealed that the heat resistance of each variant reverse transcriptase was significantly improved. C310A shown in Table 4 showed a residual activity of 93% at 45°C for 5 minutes, 70% at 50°C for 5 minutes, and 29% at 55°C for 5 minutes. It was revealed that the thermal stability was significantly improved compared with WT. From the above, it was revealed that all of the variant reverse transcriptases had improved thermal stability, and that particularly C310A significantly contributed to the improvement of thermal stability.

Example 6: Heat Resistance Test of Variant Reverse Transcriptases (C310A, C310I, and C310G)

A variant MMLV plasmid, which was designed to modify the cysteine residue at position 310 with an isoleucine residue, was produced by the same procedure as in Example 1 using pMMLV as a template and using a KOD-Plus-Mutagenesis Kit (produced by Toyobo Co., Ltd.). Then, the plasmid was transformed into BL21-CodonPlus competent cells (Agilent Technologies), and the cells were cultured in the same manner as in Example 2, thereby preparing a variant MMLV reverse transcriptase (C310I). When the reverse transcriptase activity of the thus-obtained C310I variant was measured, it was confirmed that this variant had sufficient reverse transcription activity equivalent to that of wild-type MMLV reverse transcriptase.

The C310A variant prepared in Example 2 and the above C310I variant were each diluted with a storage buffer (50 mM Tris-HCl (pH: 7.5), 300 mM KCl, 50% glycerol, 0.1 mM EDTA) to 10 U/μL, and heat-treated at 50°C for 5 minutes. Then, the reverse transcription activity was measured to determine the residual activity of each variant after heat treatment according to Formula (I) above. As a comparative example, the wild-type MMLV reverse transcriptase (WT) was similarly heat-treated at 50°C for 5 minutes, and the residual activity after heat treatment was determined.

The results indicated that the residual activity of the C310A and C310I variant reverse transcriptases after heat treatment at 50°C for 5 minutes was about 1.19 times and about 1.26 times, respectively, higher than that of the wild-type MMLV reverse transcriptase (WT), which was a comparative example. The results confirmed that the stability of the reverse transcriptases was improved not only by modifying the cysteine residue (C) at position 310 with an alanine residue (A), but also by substituting it with an isoleucine residue (I). Alanine and isoleucine are both known as non-polar amino acids having an isoelectric point of about 6.0 and exhibiting common properties. Therefore, it can be understood that particularly high stability can be obtained by modifying a cysteine residue in the MMLV reverse transcriptase with such an amino acid residue. Further, the cysteine residue at position 310 was similarly modified with a glycine residue to newly prepare a variant MMLV reverse transcriptase (C310G), and the stability under heat treatment conditions at 50°C for 5 minutes was evaluated. As a result, it was confirmed that the residual activity was higher than the residual activity of the wild-type MMLV reverse transcriptase, and that the stability was improved.

Industrial Applicability

The present invention provides a modified reverse transcriptase with excellent stability useful in the field of molecular biology, and a reagent, kit, and the like containing the reverse transcriptase. The present invention is particularly useful for gene expression analysis, and is highly versatile and highly convenient; therefore, the present invention can be used not only for research but also for clinical diagnosis, environmental tests, and the like.

**Claims**

1. A variant reverse transcriptase consisting of an amino acid sequence having at least 90% identity with an amino acid sequence represented by SEQ ID NO: 1, wherein a cysteine residue at a position corresponding to at least one position selected from the group consisting of 90, 157, 236, 262, 310, 409, 495, and 635 is modified.

2. The variant reverse transcriptase according to claim 1, consisting of an amino acid sequence having deletion, substitution, and/or addition of one or several amino acid residues in the amino acid sequence represented by SEQ ID NO: 1, wherein a cysteine residue at a position corresponding to at least one position selected from the group consisting of 90, 157, 236, 262, 310, 409, 495, and 635 is modified.

3. The variant reverse transcriptase according to claim 1 or 2, wherein the modification of the cysteine residue is deletion and/or substitution of the cysteine residue with another amino acid residue.

4. The variant reverse transcriptase according to any one of claims 1 to 3, wherein the modification of the cysteine residue is substitution with at least one non-polar amino acid residue selected from the group consisting of alanine, glycine, valine, leucine, and isoleucine.

5. The variant reverse transcriptase according to any one of claims 1 to 4, wherein the modification of the cysteine residue is substitution with an alanine residue.

6. The variant reverse transcriptase according to any one of claims 1 to 5, wherein in the amino acid sequence having at least 90% identity with the amino acid sequence represented by SEQ ID NO: 1, at least a cysteine residue at a position corresponding to position 310 is modified.

7. The variant reverse transcriptase according to any one of claims 1 to 6, wherein the modification of the cysteine residue at the position corresponding to position 310 is substitution with a non-polar amino acid residue selected from the group consisting of alanine, glycine, valine, leucine, and isoleucine.

8. The variant reverse transcriptase according to any one of claims 1 to 7, wherein the modification of the cysteine residue at the position corresponding to position 310 is substitution with an alanine residue.

9. The variant reverse transcriptase according to any one of claims 1 to 8, which lacks RNase activity.

10. A variant reverse transcriptase having a residual activity of 30% or more when stored at 25°C for 35 days.

11. A variant reverse transcriptase having a residual activity of 50% or more when stored at 25°C for 35 days.

**12.** A polynucleotide encoding the reverse transcriptase according to any one of claims 1 to 8.

**13.** A vector comprising the polynucleotide according to claim 12.

**14.** A cell transformed with the vector according to claim 13.

**15.** A reagent comprising at least one selected from the group consisting of the variant reverse transcriptase according to any one of claims 1 to 11, the polynucleotide according to claim 12, the vector according to claim 13, and the cell according to claim 14.

**16.** A method for producing the variant reverse transcriptase according to any one of claims 1 to 11 using at least one selected from the group consisting of the polynucleotide according to claim 12, the vector according to claim 13, the cell according to claim 14, and the reagent according to claim 15.

**17.** A reverse transcription method comprising synthesizing cDNA from an RNA template using the variant reverse transcriptase according to any one of claims 1 to 11.

**18.** A kit comprising the variant reverse transcriptase according to any one of claims 1 to 11.

**19.** The kit according to claim 18, for use in synthesis of cDNA using RNA as a template.

Fig. 1

Fig. 2

## INTERNATIONAL SEARCH REPORT

International application No.

PCT/JP2020/028319

### A. CLASSIFICATION OF SUBJECT MATTER

C12N 5/10(2006.01)i; C12N 1/15(2006.01)i; C12N 1/19(2006.01)i; C12N 1/21(2006.01)i; C12N 15/54(2006.01)i; C12N 15/63(2006.01)i; C12N 9/00(2006.01)i; C12N 9/10(2006.01)i

FI: C12N15/54 ZNA; C12N9/00; C12N9/10; C12N15/63 Z; C12N1/15; C12N1/19; C12N1/21; C12N5/10

According to International Patent Classification (IPC) or to both national classification and IPC

### B. FIELDS SEARCHED

Minimum documentation searched (classification system followed by classification symbols)

C12N5/10; C12N1/15; C12N1/19; C12N1/21; C12N15/54; C12N15/63; C12N9/00; C12N9/10

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

JSTPlus/JMEDPlus/JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
|---|---|---|
| X | BABA, Misato et al., "Further increase in thermostability of Moloney murine leukemia virus reverse transcriptase by mutational combination, Protein Engineering", Design & Selection,, 2017, vol. 30, no. 8, pp. 551-557, table 1, p. 552 | 1-9, 12-19 |
| A | table 1, p. 552 | 10, 11 |
| A | KONISHI, Atsushi et al., "Amino acid substitutions away from the RNase H catalytic site increase the thermal stability of Moloney murine leukemia virus reverse transcriptase through RNase H inactivation", Biochemical and Biophysical Research Communications, 2014, vol. 454, Issue 2, pp. 269-274, results | 1-19 |

☒ Further documents are listed in the continuation of Box C. ☐ See patent family annex.

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| | |
|---|---|
| Date of the actual completion of the international search | Date of mailing of the international search report |
| 26 August 2020 (26.08.2020) | 29 September 2020 (29.09.2020) |

| | |
|---|---|
| Name and mailing address of the ISA/ | Authorized officer |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2020/028319 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | KATANO, Yuta et al., "Generation of thermostable Moloney murine leukemia virus reverse transcriptase variants using site saturation mutagenesis library and cell-free protein expression system", Bioscience, Biotechnology, and Biochemistry, 2017, vol. 81, no. 12, pp. 2339-2345, table 1 | 1-19 |
| A | US 2018/0312822 A1 (10X GENOMICS, INC.) 01.12.2018 (2018-12-01) paragraphs [0191]-[0193] (Family: none) | 1-19 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000139457 A

- JP 6180002 B

**Non-patent literature cited in the description**

- *Journal of Biotechnology,* 2010, vol. 150 (3), 299-306

- Molecular Cloning. 2